Europäisches Patentamt

European Patent Office

Office européen des brevets

(11) Publication number: **0 254 845**
**A2**

## (12) EUROPEAN PATENT APPLICATION

(21) Application number: 87108214.5

(51) Int. Cl.⁴: **A61K 31/715**

(22) Date of filing: 05.06.87

(30) Priority: 10.06.86 US 872545

(43) Date of publication of application:
**03.02.88 Bulletin 88/05**

(84) Designated Contracting States:
**AT BE CH DE ES FR GB GR IT LI LU NL SE**

(71) Applicant: LESCARDEN INC.
790 Madison Avenue
New York New York 10021(US)

(72) Inventor: Farrington, George L.
370 First Avenue
New York New York 10010(US)
Inventor: Thorbecke, Jeanette G.
3719 Regatta Place
Doulgaston New York 11363(US)

(74) Representative: Patentanwälte Grünecker,
Kinkeldey, Stockmair & Partner
Maximilianstrasse 58
D-8000 München 22(DE)

(54) **Immune stimulation with chondroitin sulfate.**

(57) Methods and compositions for augmenting the immune response in a mammal or an avian comprising administration of an immune stimulating-effective quantity of chondroitin sulfate. Chondroitin sulfate is also administered in an amount sufficient to enhance the immune response resulting from vaccine or antigen administration.

EP 0 254 845 A2

## IMMUNE STIMULATION WITH CHONDROITIN SULFATE

Field of the Invention

This invention relates to compositions and methods for stimulating immune responses and augmenting normal immune responses. More particularly, this invention relates to compositions containing chondroitin sulfates and to methods using such compositions to stimulate immune responses in mammals or avians.

Background of the Invention

Chondroitin sulfate, or more accurately, chondroitin sulfates, are highly viscous, glycosaminoglycans with N-acetyl-chondrosine as the repeating unit and with one sulfate residue per disaccharide unit. The N-acetyl chondrosine unit is composed of D-glucuronic acid and N-acetylgalactosamine linked via l-3 linkages. Addition of a sulfate group to the C-4 carbon of N-acetylgalactosamine yields chondroitin 4-sulfate. Alternatively, addition of a sulfate group to the number 6 carbon of the N-acetylgalactosamine group yields chondroitin 6-sulfate. Chondroitin 4-sulfate and chondroitin 6-sulfate are the most abundant mucopolysaccharides in the bodies of mammals and occur in both cartilaginous and soft connective tissue. Naturally occurring mixtures of these sulfates have been termed chondroitin sulfate A and chondroitin sulfate C.

Extracts manufactured from cartilage have previously been employed for various biological activities. They have been used as immune modulators (Balassa, U.S. Patent No. 4,212,857), and as inhibitors of tumor vascularization (Langer, et al., Science 193 :70-72, 1976; Lee, et al., Science 221:1185-1187, 1983). It was not demonstrated, however, that these activities were due to chrondroitin sulfate. Chondroitin sulfate A and C have been used to inhibit atherosclerosis (Morrison, U.S. Patent No. 3,895,106; Morrison, U.S. Patent No. 3,895,107).

The present inventors have now discovered that chondroitin sulfate, which constitute a major component of cartilage, possess immunostimulatory activity both in vivo and in vitro. Chondroitin sulfate is a natural substance produced in the body, and it displays a low toxicity. Due to its newly discovered immunomodulatory properties, it can be employed as a "biological response modifier" (i.e., a substance which will alter the biological state of a cell - the term includes substances that enhance normal immune functions).

Currently, other substances are being studied for their capacity to enhance immune responses and their potential as treatments for neoplastic diseases. In particular, interferons and interleukin-II are examples of "natural" products being currently examined for their potential as anti-cancer agents and biological response modifiers. However, preliminary results with interferons have been disappointing and interleukin-II studies are too preliminary to evaluate at this time.

Certain glycosaminoglycans and related sulfated polysaccharide compounds have been examined for their immunomodulating properties. Palacios, et al., ( Journal of Immunology, 128:621-624, 1982) reported that dextran sulfate, a sulfated polysaccharide, was a potent mitogen for human peripheral T lymphocytes but not for adult or neonatal B lymphocytes. Ginsburg, et al. (Inflammation, 6:343-364, 1982) disclosed that dextran sulfate enhanced the bactericidal properties of leukocytes and macrophages toward both extracellular and intracellular microorganisms. Bey, et al. (Immunology, 54 :487, 1985) reported that dextran sulfate enhances the antibody response to ovalbumin in sheep. Sugawara, et al. (Microbiological Immunology, 28:831-839, 1984) reported that some polysaccharides with sulfate groups are human T-cell mitogens and murine polyclonal B-cell activators. However, this article indicated that chondroitin 4-sulfate and chondroitin 6-sulfate failed to induce significant mitogenic changes in human T cells and mouse B cells.

Frieri, et al. ( Journal of Immunology, 131:1941-1948, 1983) identified heparin as a granule-associated suppressing factor of lymphocyte blastogenesis. These investigators also reported that these findings were reproduced by using oversulfated chondroitin 6-sulfate - i.e., chondroitin-6-sulfate which has had sulfate groups added at the 4-position. Bey, et al. (Infection and Immunity, 26:64-69, 1979) disclosed the inhibitory effects of hyaluronic acid on immune reactions. Bradford et al (Cellular Immunology, 14:22, 1974) reported that heparin and dextran sulfate and other polysulfated polysaccharides cause lymphocytosis in rodents by interfering with lymphocyte recirculation.

In summary, the prior art teaches that dextran sulfate (which is not a glycosaminoglycan and is therefore different from chondroitin sulfate) has shown immunostimulatory activity in various assays. This does not mean that all sulfated polysaccharides or, in particular, all of the substances termed "glycosaminoglycans" have immunostimulatory activity, since other members of the glycosaminoglycan group, such as hyaluronic acid and heparin, inhibit certain immune responses. Chondroitin sulfate, where studied, was reported as having no effect in these assays.

It is clear from the above discussion that a more complete understanding of the nature and role of chondroitin sulfates and their interactions with the immune system is necessary. Therefore, a broad object of this invention is to increase this understanding and to use it to develop therapeutic methods for immune stimulation of a mammal or avian in need of such stimulation.

Another object of the present invention is to provide a method for increasing the efficiency of immunization protocols in mammals or avians.

Another object is to provide a method for enhancing antibody production.

Another object is to provide a convenient and safe method for stimulating the immune system and to use such a method in the management or treatment of mammals or avians in need of such stimulation.

Another object is to provide a substance that would be suitable for use as a vaccine adjuvant.

These and other objects of the present invention will be apparent to those skilled in the art in light of the present description and accompanying claims.

## Summary of the Invention

One aspect of the present invention is directed to a composition having immunostimulatory activity comprising as an active ingredient an amount of a chondroitin sulfate effective to modulate immune responses in a mammal or in avian species. A preferred form of this composition is a parenteral dosage form containing said chondroitin sulfate and a pharmaceutically acceptable carrier.

Another aspect of the present invention is directed to a method for enhancing immune responses in a mammal or avian in need of such treatment comprising administering to said mammal or avian an amount of a chondroitin sulfate effective to stimulate the immune response in said mammal or avian.

## Detailed Description of the Invention

In accordance with the present invention, chondroitin sulfates and preparations containing chondroitin sulfates can be used to stimulate immune responses in mammals or avians in need of such treatment, and to augment immune responses in normal mammals or avians challenged with an antigen (e.g., vaccinated).

The present inventors have established that chondroitin sulfate has immunostimulating properties. This was surprising because the current literature has not shown chondroitin sulfate to have significant immunostimulating effects.

The present inventors have established that the immunostimulating activity is in fact due to chondroitin sulfate by showing the following:

1. Chondroitin sulfate was effective in stimulating immune responses in a dose-dependent fashion.

2. The activity of chondroitin sulfate C, from two different commercial sources, was inhibited by chondroitinase ABC treatment but not by treatment by hyaluronidase.

3. The immune stimulation produced by chondroitin sulfate is synergistic with lipopolysaccharide (LPS), and additive with the stimulation produced by dextran sulfate. Hence, chondroitin sulfate-induced immune stimulation is not an experimental artifact. The additivity to dextran sulfate suggests that chondroitin sulfate affects a different group of immune-active cells than this polysulfated polysaccharide.

The preferred method of using chondroitin sulfate is in a parenteral liquid dosage form, preferably also comprising a pharmaceutically acceptable carrier. Appropriate chondroitin sulfate amounts for treating humans will range from 0.01 to about 1 gram per treatment, and preferably from 0.05 to 0.2 grams per treatment.

Frequency of treatments will vary according to the response of the patient to chondroitin sulfate but will generally range from single treatments to multiple treatments depending upon the severity of the disease.

To augment responses to immunization, a preferred regimen is one injection comprising from about 0.05 grams to about 0.2 grams of chondroitin sulfate as an adjuvant in a preparation also comprising the immunization agent. If desired, however, chondroitin sulfate can be administered before or after the immunization agent, and/or together with additional adjuvants, carriers or diluents.

The number of treatments will vary according to the condition to be treated and might also vary from individual to individual. When used as a biological response modifier, e.g. to combat immune deficiencies of the aged, or help in the defense against infections or tumors, additional treatments will be necessary at appropriate intervals as determined by routine experimentation. Administration of up to 400 mg/kg of chondroitin sulfate is not toxic to mice.

Preferred carriers for chondroitin sulfate-containing compositions include but are not limited to the following: buffered saline, balanced physiological salt solutions, etc.

The present invention is further described below by reference to specific examples, which are intended to illustrate the present invention without limiting its scope.

Source of Chondroitin Sulfate

Chondroitin sulfate A is an 80:20 mixture of chondroitin 4-sulfate : chondroitin 6-sulfate, and chondroitin sulfate C is a 10:90 mixture of 4-sulfate : 6-sulfate. Supplies were obtained from Sigma Chemical Co. (St. Louis, MO) and Miles Laboratory (Research Division, Elkhart, IN).

Example I: Activity of Chondroitin Sulfate In Vivo in Augmenting the PFC Response to SRBC

The ability of chondroitin sulfate to augment the antibody response of mice to a specific antigen was tested in vivo. The spleens of $(SJL \times Balb/c)F_1$ mice [bred from mice supplied by Jackson Laboratories (Bar Harbor, ME) and Charles River Breeding Laboratories (Wilmington, MA)], were assayed for plaque-forming cells (PFC) four to five days after intravenous (i.v.) injection of antigen (sheep red blood cells (SRBC) (Colorado Serum Company, Denver, CO)) with and without a concomitant intrapertoneal (i.p.) injection of chondroitin sulfate. The assay was as follows: single-cell suspensions were prepared from individual mouse spleens. The cells were suspended in Hanks' Balanced Salt Solution (HBSS, GIBCO, Grand Island, NY) and washed. Direct PFC were enumerated using the well-known method of Jerne, N.K., et al. (in Cell-Bound Antibody, Amos, E. and Kaprowsky, H., Eds., Wistar Institute Press, pp. 109-III, 1983) with the slide modification of Mishell, R.I. and Dutton, R.W. (J.Exp. Med. 126:423, 1967), both incorporated by reference.

The results are presented below in Table I. The numerals in parentheses denote the number of animals per group.

### Table 1

| Strain | $(SJL \times Balb/c)F_1$ |
|---|---|
| SRBC, dose, route | $2 \times 10^6$, i.v. |
| None (control) | 735 $x/\div$ 1.2a(5) |
| Hyaluronic acid, 5 mg, i.p. | 665 $x/\div$ 1.2(3) |
| Chondroitin sulfate C, 5 mg, i.p. | 3,610 $x/\div$ 1.2(5) |
| 50 mg, i.p. | 780 $x/\div$ 1.4(3) |

aGeom. Mean $x/\div$ SE of PFC/spleen, four days after injections of SRBC.

As can be seen in Table I, chondroitin sulfate C at 5 mg, led to a five-fold stimulation in the PFC response to SRBC over control values. Hyaluronic acid at the same concentration did not lead to this stimulating effect. At a very high chondroitin sulfate dose (50 mg) there is no augmentation of the response to antigen. This pattern of response is also found in vitro (see Table 3 below).

The effect of chondroitin sulfate A or C on the PFC response was further analyzed. In the experiments described below in Tables 2 and 3, antigen injected was either SRBC or TNP-Ficoll (TPN-F). TNP-F is trinitrophenyl (Sigma, St. Louis, MO) covalently bonded to Ficoll (40,000 MW, Pharmacia Fine Chemicals, Piscataway, NJ) by the method of Mitchell, G. F., et al. (Eur. J. Immunol., 2:460, 1972, incorporated by reference) and is a T-cell independent antigen. In addition, three different strains of mice were utilized: $CB6F_1$ (Charles River Breeding Laboratories, Inc., Wilmington, MA), $(SJL \times Balb/c)F_1$ or Balb/c (Charles River Breeding Laboratories, Inc., Wilmington, MA). PFC responses were analyzed as in Table I above.

## Table 2

| Expt.* | Antigen Injected | Adjuvant** Injected | Geom. mean SE(n) of PFC/Spleen | p.vs. Control |
|---|---|---|---|---|
| 1 | $10^6$ SRBC | None | 1,320 x/+ 1.1 (10) | — |
|  | $10^6$ SRBC | 5 mg ChS.A | 1,700 x/+ 1.8 (3) | N.S.+ |
|  | $10^6$ SRBC | 1 mg ChS.C | 1,810 x/+ 1.2 (3) | N.S |
|  | $10^6$ SRBC | 5 mg ChS.C | 3,610 x/+ 1.3 (3) | N.S. (p=.169) |
|  | None | 5 mg ChS.C | 290 x/+ 1.2 (5) | p = 0.0002 |
| 2 | 2 x $10^6$ SRBC | None | 1,050 x/+ 1.2 (9) | — |
|  | 2 x $10^6$ SRBC | 5 mg ChS.A | 5,400 x/+ 1.5 (4) | p = 0.050 |
|  | 2 x $10^6$ SRBC | 10 mg Chs.A | 3,670 x/+ 1.5 (4) | p = 0.0077 |
|  | 2 x $10^6$ SRBC | 5 mg ChS.C | 17,050 x/+ 1.5 (4) | p = 0.010 |
|  | 2 x $10^6$ SRBC | 10 mg ChS.C | 22,660 x/+ 1.1 (4) | p = 0.0001 |
| 3 | 0.2mcg TNP-F | None | 24,060 x/+ 1.2 (5) | — |
|  | 0.2mcg TNP-F | 5 mg ChS.C | 40,620 x/+ 1.4 (5) | N.S. (p= 0.305) |
|  | 0.2mcg TNP-F | 5 mg ChS.C (Day 2) | 49,420 x/+ 1.1 (5) | p = 0.0062 |

*CB6F$_1$ mice (Expt. 1), (SJL x Balb/c) F$_1$ mice (Expt. 2) and Balb/c mice (Expt. 3).

**Chondroitin sulfate (ChS.) was injected i.p. at the same time as antigen unless otherwise noted.

+N.S. Not Significant.

In CB6F$_1$ mice, chondroitin sulfate C, at 5 mg caused a 2-3 fold stimulation in the PFC response over control values. In (SJL x Balb/c)F$_1$ mice injected with 2 x $10^6$ SRBC i.p., both chondroitin sulfate A and C stimulated antibody production. Chondroitin sulfate A caused a 5-fold stimulation in the PFC response at 5 mg, while chondroitin sulfate C produced a 22-fold increase when injected at 10 mg (Table 2).

Table 3

| Expt. | Antigen Injected | Adjuvant Injected i.v. | Geom. Mean x/÷ SE (n) of PFC/spleen | P vs. Control | Reciprocals of Serum Titers log$_2$ ± SE |
|---|---|---|---|---|---|
| 1 | 2x10$^6$ SRBC | None | 1,600 x/÷ 1.2 (5) | – | 6.1 + 0.4 |
|  | 2x10$^6$ SRBC | 10 mg ChS.C. | 8,400 x/÷ 1.9 (4) | NS(p=.15) | 8.1 + 0.8 |
|  | 2x10$^6$ SRBC | 0.5 mg ChS.C. | 9,400 x/÷ 1.2 (4) | p=.0006 | 8.0 + 0.4 |
|  | 2x10$^6$ SRBC | 0.05 mg ChS.C. | 15,200 x/÷ 1.2 (4) | p=.0001 | 9.5 + 0.3 |
|  | 2x10$^6$ SRBC | 0.005 mg ChS.C. | 1,700 x/÷ 1.3 (4) | NS | 5.5 + 0.9 |
| 2 | 2x10$^6$ SRBC | None | 3,540 x/÷ 1.1 (4) | – | 6.3 + 0.6 |
|  | 2x10$^6$ SRBC | 5 mg ChS.C. | 25,800 x/÷ 1.1 (4) | p=.0001 | 8.8 + 0.3 |
|  | 2x10$^6$ SRBC | 0.5 mg ChS.C. | 42,970 x/÷ 1.2 (4) | p=.0001 | 9.0 + 0.4 |
|  | 2x10$^6$ SRBC | 0.05 mg ChS.C. | 50,750 x/÷ 1.3 (4) | p=.0004 | 9.5 + 0.3 |
|  | 2x10$^6$ SRBC | 0.5 mg ChS.C.** | 23,100 x/÷ 1.5 (4) | p=.04 | 9.0 + 0.4 |
| 3 | 10 mcg TNP-F | None | 38,900 x/÷ 1.1 (5) | – | |
|  | 10 mcg TNP-F | 0.5 m g ChS.C. | 42,700 x/÷ 1.1 (5) | NS(p=.416) | |
|  | 10 mcg TNP-F | 0.5 m g ChS.C. (Day 2)*** | 71,600 x/÷ 1.1 (5) | p=0.11 | |

*Balb/c mice (Expts. 1 and 3), (SJL x Balb/c) F$_1$ mice (Expt. 2) and Balb/C mice (Expt. 3). PFC were determined on day 5 (Expts. 1 and 2) or day 4 (Expt. 3).

**Chondroitin sulfate (ChS.C.) was injected i.v. except in this case, where it was injected as 0.25 mg in each of the front footpads.

***ChS.C. was injected at the same time as antigen except in this case, where it was injected on day 2.

The effect of chondroitin sulfate on the antibody response was higher and detectable at lower dosages of chondroitin sulfate when it was injected intravenously than when given intraperitoneally. (Compare Tables 2 and 3.) There also was a significant effect when chondroitin sulfate was injected subcutaneously (in front footpads). Serum hemagglutinin titers (done on serial dilutions of sera in microtiter plates) corresponded very well to the numbers of PFC per spleen (Table 3).

Example II: Comparison Between Different Chondroitin Sulfates In Vitro

The effect of different chondroitin sulfates on the incorporation of tritiated-thymidine into mouse lymphoid cells, a marker for lymphocyte proliferation, was studied in the presence or the absence of a mitogen, lipopolysaccharide (LPS). Mouse Balb/c spleen cells were obtained by teasing the spleens into single cell suspensions. Spleen cells were cultured in 96-well culture dishes (Falcon #3040) at 2×10$^5$ cells/ml in 0.2 ml tissue culture medium [RPMI 1640 (Gibco, Grand Island, NY) containing 10% fetal calf serum, antibiotics, and 5 × 10$^{-5}$ M 2-mercaptoethanol]. Endotoxin (LPS, Difco, E. coli B., 10 mcg/ml) was added to some of the cultures. After 72 hours of culture at 37°C in an atmosphere of 5% CO$_2$ in air, each well received 0.5 microCi of $^3$H-thymidine (1.9 Ci/mM, New England Nuclear, Boston, MA). Cultures were harvested 6-8 hours later and the incorporated radioactivity was determined.

## Table 4

### IN VITRO RESPONSE OF Balb/c SPLEEN CELLS TO CHONDROITIN SULFATE C

**I.**

| Added to Culture | Concentration (mg) | % of Control cpm $^3$H-thymidine incorporation | |
|---|---|---|---|
| | | without LPS 100%=1,247+189 | with LPS 100%=62,141+3,364 |
| | 0.0 | | |
| ChS.C (Miles) | 0.1 | 84 | 122 |
| | 0.5 | 297 | 126 |
| | 2 | 299 | 166 |
| | 5 | 801 | 169 |
| ChS.C (Sigma) | 0.1 | 361 | 148 |
| | 0.5 | 381 | 163 |
| | 2 | 636 | 190 |
| | 5 | 565 | 187 |

**II.**

| | Concentration (mg) | without LPS 100%=1,819+523 | with LPS 100%=35,687+3,484 |
|---|---|---|---|
| | 0.0 | | |
| ChS.C (Sigma) | 5 | 330 | 225 |
| | 2 | 682 | 272 |
| | 1 | 645 | 217 |
| | 0.2 | 290 | 174 |
| | 0.05 | 209 | 120 |
| | 0.01 | 112 | 122 |
| | 0.002 | 159 | 125 |

As can be seen in Table 4, two different chondroitin sulfates demonstrated enhancement of incorporation of tritiated thymidine into lymphocytes both in the presence and in the absence of LPS. This enhancement appeared to be greater between the concentrations of I and 5 mg in both the presence and the absence of LPS.

In order to expand the above results, lymph node (LN) or spleen cells were obtained from athymic (nude) mice (Division of Cancer Treatment Animal Program, National Cancer Institute) and incubated with either chondroitin sulfate A or C for 96 hours at 37°C in the presence or absence of LPS. The results are shown below in Table 5.

## Table 5

### STIMULATION OF SPLEEN AND LYMPH NODE CELLS FROM ATHYMIC nu/nu MICE BY CHONDROITIN SULFATES A AND C

| Responder Cells | Chondroitin Sulfate Added to Cultures | | 3H-Thymidine incorporation (cpm)* | |
|---|---|---|---|---|
| | Type | mg/ml | Without LPS | With 10 mcg/ml LPS |
| LN | A | 4 | 4,865 | 61,243 |
| | | 1 | 3,476 | 59,164 |
| | | 0.4 | 1,971 | 52,414 |
| | C | 4 | 10,104 | 66,015 |
| | | 1 | 7,743 | 82,696 |
| | | 0.4 | 5,243 | 71,612 |
| | | 0 | 0* | 47,909 |
| Spleen | A | 4 | 12,219 | 38,462 |
| | | 1 | 5,753 | 21,161 |
| | | 0.4 | 3,850 | 18,440 |
| | C | 4 | 25,871 | 41,096 |
| | | 1 | 20,044 | 40,832 |
| | | 0.4 | 9,672 | 17,623 |
| | | 0 | 0* | 19,820 |

*Subtracted background CPM was 3309 ± 136 for LN and 8534 ± 537 for spleen cells.

Chondroitin sulfates A and C caused a significant increase in 3H-thymidine incorporation in lymph node and spleen cells in the absence of LPS in a dose-dependent manner. The stimulation was greatest in spleen cells with 4 mg/ml chondroitin sulfate C. The effect was also observed in the presence of LPS, with the effects of the two agents being either additive or synergistic. The above experiment demonstrates that this effect is a T-cell-independent phenomenon, as the source of cells in Table 5 was from athymic nude mice, which are deficient in T cells. These results were reproduced below in Table 6 using lymphocytes from normal Balb/c mice, where the optimal doses of chondroitin sulfates A and C were found to be 2.5-5 mg/ml in the presence or absence of LPS (underlined values in Table 6).

## Table 6

### % of control cpm 3H-thymidine incorporation

| Added Dose of ChS.C. (mg/ml) | Without LPS | | With LPS | |
|---|---|---|---|---|
| None (Control) | 100%=929 ± 27 | | 100%=27,650 ± 634 | |
| | ChS. A | ChS. C | ChS. A | ChS. C |
| 10 | 44 | 376 | 145 | 2 |
| 5 | 370 | 828 | 182 | 158 |
| 2.5 | 346 | 851 | 149 | 231 |
| 1.3 | 65 | 711 | 157 | 208 |
| 0.8 | 157 | 570 | 127 | 160 |
| 0.4 | 172 | 561 | 133 | 166 |
| 0.2 | 115 | 347 | 126 | 163 |
| 0.1 | 123 | 253 | 129 | 157 |

Example III: Effect In Vitro of Digested and Undigested Chondroitin Sulfate C

To demonstrate the specificity involved in this immune stimulation, chondroitin sulfate C was evaluated for its immunostimulating potential both with and without preincubation with chondroitinase ABC, an enzyme that degrades chondroitin sulfate and with and without preincubation with hyaluronidase, an enzyme which specifically degrades hyaluronic acid (obtained from Sigma, St. Louis, MÓ). The results are presented below in Table 7.

## Table 7

### EFFECT OF PREINCUBATION WITH CHONDROITINASE[1] ABC OR HYALURONIDASE ON THE ABILITY OF CHONDROITIN SULFATE C TO STIMULATE MURINE SPLEEN CELLS IN VITRO

| Expt. | Chondroitin Sulfate C | Enzyme Treatment of Chondroitin Sulfate | $^3$H-thymidine incorporation (cpm)* | | | |
|---|---|---|---|---|---|---|
| | | | Without LPS | p vs control | With LPS | p vs control |
| 1 | 2 mg | Incubation w/o enz. | 14,247 | — | 132,871 | — |
| | 0.5 mg | " | 8,851 | — | ND | ND |
| | 2 mg | Chnase ABC (24 hr) | 4,913 | 0.0001 | 114,944 | NS |
| | 0.5 mg | (1 u/20 mg) | 3,991 | 0.0001 | ND | ND |
| 2 | 2 mg | Incubation w/o enz. | 9,135 | — | 145,637 | — |
| | 0.5 | " | 7,382 | — | ND | ND |
| | 2 mg | Chnase ABC (4 hr) | 5,446 | 0.0068 | 113,692 | 0.0010 |
| | 0.5 mg | (1 u/20 mg) | 3,150 | 0.0014 | ND | ND |
| 3 | 2 mg | None | 45,368 | — | 120,325 | — |
| | 0.5 mg | None | 21,162 | — | 129,246 | — |
| | 2 mg | Chnase ABC (1hr) | 26,843 | 0.0002 | 104,403 | NS |
| | 0.5 | (1 u/20 mg) | 11,585 | 0.0003 | 76,741 | 0.0001 |
| 4 | 4 mg | None | 8,543 | — | ND | ND |
| | 0.8 | None | 7,586 | — | 117,345 | — |
| | 4 mg | Hyaluronidase (3 hr) | 6,950 | NS | ND | ND |
| | 0.8 mg | (2 mg/80 mg) | 8,642 | NS | 104,457 | NS |
| 5 | 0.8 mg | None | 11,747 | — | 112,664 | — |
| | 0.8 mg | Hyaluronidase (13 hr) (2 mg/80 mg) | 12,471 | NS | 110,728 | NS |

*The relevant background CPM (+ Enzyme) were subtracted. They were 2000-3000 CPM with or without enzyme. The control response to LPS alone was approximately 80,000 CPM in each of these experiments. Each value represents the mean of quintuplate determinations.

ND - Not Done: NS - Not Significant

[1] Abbr. "Chnase"

As can be seen in the above data, chondroitin sulfate C was able to stimulate the immune response in a dose-dependent fashion both in the presence and in the absence of LPS. It should be noted that the stimulatory effect was much greater in the absence of LPS than in its presence. Predigestion with chondroitinase ABC led to a complete loss in this immunostimulating activity both in the presence and absence of a co-mitogen.

To confirm the specificity of this reaction, chondroitin sulfate alone was incubated with hyaluronidase for various periods of time before addition to the culture medium. (Expts. 4 and 5) The data are presented in Table 7. These data show that treatment of chondroitin sulfate with hyaluronidase did not diminish the ability of the former to enhance this specific immune response.

Example IV: Synergy Between Chondroitin Sulfate and LPS and Dextran Sulfate in Direct Polyclonal Activation of B-Cells

An embodiment of the present invention comprises the co-administration of chondroitin sulfate with a co-mitogenic agent. The effect of LPS and chondroitin sulfate on direct polyclonal activation of mouse B cells was examined. The data are presented below in Table 8.

## Table 8

### ADDITIVE EFFECT OF VARIOUS B CELL STIMULI ON PROLIFERATION OF Balb/c SPLEEN CELLS IN VITRO

$^3$H–Thymidine incorporation (cpm) in presence of:

| Expt. No. | Added Stimulant | None | ChS.C* | ChS.A* | DXS+ |
|---|---|---|---|---|---|
| 1 | None | 0 | 6,981 | 2,510 | ND |
|   | LPS | 26,721 | 62,835 | 49,449 | ND |
| 2 | None | 0 | 10,596 | ND | 27,370 |
|   | LPS | 33,868 | 95,202 | ND | 106,107 |
| 3 | None | 0 | 24,193 | ND | 8,499 |
|   | LPS | 77,474 | 132,781 | ND | 124,588 |
| 4 | LPS | 65,585 | 154,720 | ND | 153,292 |
|   | ChS.C | 24,572 | 24,284 | ND | 43,416 |
|   | DXS | 13,800 | 43,416 | ND | 12,772 |
| 5** | None | 0 | 20,044 | 12,219 | 14,493 |
|   | LPS | 19,820 | 40,832 | 38,462 | ND |
|   | CHS.C | 20,044 | 25,871 | ND | 28,874 |

*Chondroitin Sulfate A was used at 4 to 5 mg/ml. Chondroitin Sulfate C was used at 2 mg (Expts. 1, 2 and 3) or 1 mg (Expts. 4 and 5) per ml.

**Spleen cells from athymic nu/nu Balb/c mice used in this experiment. In all other experiments unfractionated spleen cells from normal Balb/c mice were used.

+Dextran Sulfate (Sigma Chemical Co., St. Louis, MO) was used at 20 mcg (Expts. 1, 2, 3 and 5) or 10 mcg (Expt. 4) per ml.

From the data in Table 8, it can be seen that the co-administration of LPS with chondroitin sulfate C at either I or 2 mg per ml led to a synergistic effect in the direct activation of polyclonal B cells. A similar effect was noted with chondroitin sulfate A at 4 to 5 mg/ml. In addition, chondroitin sulfate C also demonstrated an additive effect when co-administered with dextran sulfate. These results were reproduced using spleen cells from athymic nude mice (Expt. 5, Table 8).

Example V : Chondroitin Sulfate-Mediated Immunostimulation in Chickens

In order to determine whether the immunoaugmenting properties of chondroitin sulfate C were limited to mammals, or could perhaps also be detected in the avian species, experiments were performed on chickens of the SC strain, purchased as fertilized eggs from Hy-Line Internatl. (Dallas Center, IO). Chickens were hatched in a Petersime Model 5 incubator (Gettysburg, OH) and raised first in brooders and then in cages from the age of eight weeks until use. Methods for determination of PFC were similar to those described in Example I.

The results in Table 9 below show that the intravenous injection of chondroitin sulfate simultaneously with SRBC significantly raised the PFC response in the spleens of 7-month old male chickens determined four days after antigen injection.

## Table 9

| Antigen Injected | Adjuvant Injected | Geom. Mean SE (n) of PFC/spleen | P vs Control |
|---|---|---|---|
| $5x10^7$ SRBC | None | 57,600 $^x/_+$ 1.4 (10) | -- |
| $5x10^7$ SRBC | 1 mg ChS.C. | 247,200 $^x/_+$ 1.2 (9) | p=.008 |

This result demonstrates that the adjuvant effect of chondroitin sulfate is applicable to immunization of the avian as well as the mammalian species. When IO mg chondroitin sulfate was injected, the PFC response obtained were somewhat lower than those seen after I mg.

In summary, the above data demonstrate the biological response modifying action of chondroitin sulfates A and C and their potential utility as immunostimulating agents in mammals and avians. Proliferative B-cell responses were enhanced in the absence of co-mitogens, and, in addition, these glycosaminoglycans demonstrated synergistic effects when co-administered with two different stimuli: dextran sulfate and LPS.

The invention has been described above by reference to preferred embodiments. It is understood that many additions, deletions and modifications will be apparent to one of ordinary skill in the art in light of the present description without departing from the scope of the invention, as claimed below.

## Claims

1. A composition for enhancing the immune response in a mammal or avian in need of such treatment comprising an effective amount of chondroitin sulfate for enhancing said immune response and a pharmaceutically acceptable carrier or diluent.

2. The composition of claim 1 wherein said chondroitin sulfate is contained in a parenteral dosage form.

3. The composition of claim 2 wherein said amount is effective for augmenting the immune response to an antigen.

4. The composition of claim 3 wherein said amount is effective for augmenting the immune response to a vaccine.

5. The composition of claim 1 wherein said amount is effective in mounting an immune response against a tumor in said mammal.

6. The composition of claim 5 wherein said amount is effective in reducing the tumor burden in a mammal in need of such treatment.

7. The composition of claim 1 wherein said carrier is buffered saline, or other physiological acceptable salt solution.

8. The composition of claim 6 further comprising an antigen in an amount effective for immunization of said mammal or avian.

9. The composition of claim 1 in a form suitable for parenteral administration.

10. The composition of claim 8 wherein said amount ranges between about 0.05 mg and about 2 g per injection of said composition for mice.

11. The composition of claim 9 , wherein said amount ranges between about 0.2 mg and about 10 g per injection of said composition for man.

12. The composition of claim 11 wherein said amount ranges from about 0.05 g and about 2 g per injection of said composition for man.

13. The composition of claim 9 wherein said amount ranges between about 0.05 mg and about 2 g per injection of said composition for an avian.

14. The composition of claim 1 in a form suitable for oral administration.